# EUROPEAN PATENT APPLICATION

(11) **EP 4 464 305 A1**
(43) Date of publication of application: **20.11.2024**
(21) Application number: 23461586.2
(22) Date of filing: 14.05.2023
(51) Int. Cl.: A61K 8/02, A61K 8/73

(54) **A MULTI-COMPONENT HYDROGEL SHEET FOR SKIN REGENERATION AND A METHOD OF PRODUCTION THEREOF**

(71) Applicant: Uniwersytet Zielonogórski, 65-417 Zielona Góra (PL)
(72) Inventor: Kaczmarek-Pawelska, Agnieszka, 65-119 Zielona Gora (PL); Leksycki, Kamil, 67-100 Nowa Sol (PL)
(74) Representative: JD&P Patent Attorneys Joanna Dargiewicz & Partners

(57) **Abstract**

The object of the present invention is a method of production of a multi-component hydrogel sheet containing: 2-3% by weight of sodium alginate, 0.1-1 by weight lactobionic acid, 0.75-5% by weight D-panthenol, 0.02-0.1% by weight of hyaluronic acid, 87.9-96.13% by weight deionised water; 1-3% by weight of calcium lactate as a crosslinking agent, which includes the following stages: (a) mixing sodium alginate, lactobionic acid and hyaluronic acid in powder form, preferably for 1 minute at 100 rpm; (b) mixing with distilled water, preferably at 200 rpm, and slowly adding the mixed powder ingredients using a stirrer submerged to at least half the height of the water column; (c) increasing the stirring speed, preferably to 500 rpm, adding D-panthenol drop by drop and stiring the whole for 15-30 minutes; (d) soaking two sheets of blotting paper with an aqueous solution of calcium lactate and placing the soaked sheets of blotting paper between the rollers; (e) placing the solution obtained in steps (a) to (c) between two sheets of blotting paper soaked in step (d) placed between the rollers, the rollers being spaced at between 1 and 5 mm; (f) immersion of the tissue sheets with the solution obtained in steps (a) to (c) as a precursor to the hydrogel sheet in a calcium lactate solution for a period of 20 to 30 minutes and then in distilled water, preferably for a period of 5 minutes; (g) removal of tissue paper sheets from the resulting hydrogel sheet.

## Description

The present invention relates to a multi-component hydrogel sheet for skin regeneration and a method of production thereof.

Hydrogel materials are formed by cross-linked polymer networks that contain hydrophilic groups in their structure. Their advantage is their ability to swell when reacting with water. The use of hydrogels in regenerative medicine as well as in biomedical applications has been known for many years. The first use was in 1960 in clinical trials. The first medical field in which hydrogels found application was ophthalmology. Research began on contact lenses and intraocular lenses. Already at that time, the positive effects of hydrogel materials were observed, which, due to their high oxygen permeability and lack of irritation, made it possible to eliminate inflammation and foreign body reactions in comparison with other artificial materials used previously. Such properties of hydrogels as oxygen permeability, high water content and the ability to protect against harmful external influences, e.g. mechanical, allow the materials in question to be widely used as hydrogel dressings for wound healing. In addition, hydrogels have found applications in controlled drug release systems and for tissue regeneration and cellular scaffolding.

Both hydrogels based on natural and synthetic polymers are used in regenerative medicine and cosmetology. Hydrogels based on natural polymers are created from polymers such as collagen, chitosan, agar or alginate. These are more beneficial because they produce biodegradable products during decomposition, e.g. amino acids, saccharides, unlike synthetic polymers. In contrast, hydrogels formed by synthetic polymers are characterised by their high swelling capacity and water content, wide elasticity for mechanical properties.

One of the best-known natural polymers used for research and analysis aiming to develop a biodegradable material is alginate. It is used to formulate and mix with various nanomaterials to improve the behaviour and properties of these materials. In addition, alginate is used as an emulsifier, an additive for medicines and materials used for wound healing [2, 11]. Alginate is an attractive, naturally occurring anionic and hydrophilic polysaccharide that contains blocks of β-D-mannuronic acid (M) and α-L-guluronic acid (G). The cross-linking of alginate most often takes place through ionic processes. Alginate is readily soluble in water or culture medium and is gelled using divalent cations such as Ca²⁺, Mg²⁺ or Fe²⁺. Of these cations, Ca²⁺ is the most widely used for ionic cross-linking of alginate. Calcium chloride (CaCl₂) has been shown to be one of the best sources of Ca²⁺ when performing the crosslinking procedure [2, 11].

**Table 1. Summary of the composition of alginate-based hydrogels along with their application.**

| **Item** | **Material** | **Application** | **Ref.** |
|---|---|---|---|
| 1. | Sodium alginate, aloe vera. Crosslinking agent CaCl₂. | Tissue engineering, skin tissue regeneration. | [8] |
| 2. | Sodium alginate, bovine serum albumin (BSA). Crosslinking agent CaCl₂. | Regeneration of oral tissue. | [2] |
| 3. | PVA/alginate composite and PVA/alginate composite with added bio glass (BG). Crosslinking agent CaCl₂. | Scaffolding used in biomedical engineering. | [11] |
| 4. | Silk/sodium alginate composite. Crosslinking agent CaCl₂. | Soft tissue regeneration. | [14] |
| 5. | Alginate/aloe film. Crosslinking agent CaCl₂. | Tissue regeneration, wound healing. | [9] |
| 6. | Biodegradable scaffold of a chitosan/alginate mixture. Crosslinking agent CaCl₂. Cylindrical samples 13 mm in diameter and 3 mm thick. | Tissue engineering. | [7] |
| 7. | Material consisting of hydroxyapatite, chitosan and sodium alginate. Crosslinking agent CaCl₂. | Material used for implantation. | [13] |
| 8. | Sodium alginate | Drug delivery systems. | [1] |
| 9. | Sodium alginate. Cells and antibiotics. Cross-linking agent CaCl₂. | Scaffolds for repairing damaged tissue, tissue engineering. | [5] |
| 10. | Sodium alginate, hydroxyapatite, silica. | Fibrous dressings: | [15] |
| | Crosslinking agent CaCl₂. | alginate fibre dressings, alginate/hydroxyapatite composite fibre dressings, alginate/silica composite fibre dressings). | |
| 11. | Sodium alginate, chitosan, glycerol, crosslinker CaCl₂. | Scaffolds based on alginate, chitosan and hybrid fibres with and without glycerol for tissue engineering applications. | [4] |
| 12. | Sodium alginate, chitosan, hydroxyapatite. Crosslinking agent CaCl₂. Drug: lidocaine. | The aim of the study was to determine the degradation of alginate, chitosan, hydroxyapatite composite materials with drug-adsorbed lidocaine. | [3] |
| 13. | Sodium alginate. Crosslinking agent CaCl₂. Drugs: isoniazid, rifampicin, pyrazinamide. | The aim of the study was to evaluate alginate microcapsules with attached therapeutic substances, whose degradation with drug release was analysed in simulated intestinal and gastric fluids. In vivo studies were conducted on guinea pigs. | [10] |
| 14. | Sodium alginate, chitosan, tranexamic acid (anti-hemorrhagic effect). Crosslinking agent CaCl₂. | Chitosan/alginate/terexamic acid microparticles used to develop powdered haemostatic agents with anti-haemorrhage activity for medical applications. | [6] |
| 15. | Sodium alginate, gelatine, bio-glasses. Crosslinking agent CaCl₂ | Alginate/gelatin/biofibre composites as scaffolds used in bone tissue engineering. | [12] |

Chinese patent application CN109966209A discloses a high softness make-up milk containing the following ingredients in parts by weight: 0.3-0.7 parts ascorbic acid, 1-3 parts sodium sorbate, 0.2-0.5 parts flavouring, 1-4 parts excipients, 10-15 parts softening materials. Calcium acyl lactate is used as an excipient. Sodium alginate is used as a thickener, which is mixed with guar gum and hydroxyethylcellulose.

US patent application US2021369574A1 relates to an antibacterial eyelid cleansing composition comprising (a) a hypoalcoholic acid; (b) one or more surfactants with an HL8 value of 5 to 18; and (c) an aqueous carrier, the aqueous carrier comprising (i) one or more tonicity adjusters; (ii) one or more osmolarity adjusters; and (iii) one or more pH adjusters to maintain the pH of the composition between about 4.5 and about 8.0. The composition may facilitate long-term eyelid hygiene by removing excess oil, debris and exfoliated skin and provide relief from ocular inflammation.

The antibacterial composition contains 0.01-5% by weight of one or more ingredients selected from the group consisting of propylene glycol, glycerine, liquid polyols, nut oils and derivatives, floral extracts, fruit extracts, sodium alginate, hyaluronic acid, diglycerides, triglycerides, PEG-75 Lanolin, mineral oil, silicone oil and mixtures thereof.

International patent application WO2012003704A1 relates to a moxifloxacin slow-release microsphere that is cross-linked with sodium alginate and a method of preparing the microsphere. The microsphere contains moxifloxacin, a drug carrier, an adsorbent, a reinforcing agent and a clotting agent, the drug carrier being sodium alginate, the adsorbent being albumin prepared from human plasma or bovine serum albumin, the reinforcing agent being gelatin or hyaluronic acid and the clotting agent being a divalent metal cation selected from calcium salt or barium salt.

The data available in literature, indicate a very specialised use of alginate-based hydrogels. From the point of view of medical science and tissue regeneration, this is obviously desirable. On the other hand, sodium alginate-based hydrogels can also be a carrier of skin nourishing substances, especially for sensitive and shallowly vascularised skin. In the absence of solutions in which alginate hydrogels contain active ingredients that have a positive effect on the skin, research was undertaken to develop a multicomponent hydrogel sheet for skin care.

The aim of the present invention was to develop a multi-component hydrogel sheet for skin regeneration with a unique chemical composition (content of active ingredients), varied shape and thickness, and a method for its production.

This objective was achieved by the hydrogel sheet and the method according to the present invention.

Thus, the subject matter of the present invention is a method of production of a multi-component hydrogel sheet containing: 2-3% by weight of sodium alginate, 0.1-1 by weight lactobionic acid, 0.75-5% by weight D-panthenol, 0.02-0.1% by weight of hyaluronic acid, 87.9-96.13% by weight deionised water; 1-3% by weight of calcium lactate as a crosslinking agent, which includes the following stages: (a) mixing sodium alginate, lactobionic acid and hyaluronic acid in powder form, preferably for 1 minute at 100 rpm; (b) mixing with distilled water, preferably at 200 rpm, and slowly adding the mixed powder ingredients using a stirrer submerged to at least half the height of the water column; (c) increasing the stirring speed, preferably to 500 rpm, adding D-panthenol drop by drop and stiring the whole for 15-30 minutes; (d) soaking two sheets of blotting paper with an aqueous solution of calcium lactate and placing the soaked sheets of blotting paper between the rollers; (e) placing the solution obtained in steps (a) to (c) between two sheets of blotting paper soaked in step (d) placed between the rollers, the rollers being spaced at between 1 and 5 mm; (f) immersion of the tissue sheets with the solution obtained in steps (a) to (c) as a precursor to the hydrogel sheet in a calcium lactate solution for a period of 20 to 30 minutes and then in distilled water, preferably for a period of 5 minutes; (g) removal of tissue paper sheets from the resulting hydrogel sheet.

The properties of hydrogels mainly depend on the direction of cross-linking and the orientation of the polysaccharide chains relative to each other. The direction and orientation is primarily influenced by the hydrogel production process. It depends on how the polymer is mixed with the crosslinking agent and the stand used. A method of forming alginate hydrogels by crosslinking with divalent calcium ions, usually derived from calcium chloride, has been described in the literature. Alginate hydrogels can be formed as beads, sheets or scaffold. Calcium chloride, which is most commonly used to crosslink alginates, is a substance approved as E509, but in the higher concentrations necessary to crosslink alginate, it can be irritating to the skin, as the chloride ions from calcium chloride dissociation remain as a residue in the alginate solution and can react with sensitive skin, causing burning and irritation. The available results in the literature mainly indicate the implantation of alginate hydrogels inside body shells or on wounds. The inside of the body and the skin wound are rich in chloride ions, which are derived from physiological fluids. Continuous skin tissue, on the other hand, does not contain as much chloride ions on its surface as the inside of the body. Therefore, in the study, calcium ions were extracted from calcium lactate, which is known by the symbol E327 and whose dissociation products do not show skin irritation.

The active ingredients studied were:
1) Lactobionic acid belongs to the group of bionic acid. This group is a group of chemical compounds formed by the simple oxidation of disaccharides (e.g. lactose, maltose, sucrose). Lactobionic acid is obtained by oxidation of lactose. It promotes the production of collagen and elastin, has an exfoliating effect and also supports repair processes. As a result, it smooths and softens the epidermis, reduces the thickness of the horny layer of the skin and reduces superficial wrinkles. In addition, it regenerates the skin and strengthens its barrier. The application of products containing lactobionic acid helps to reduce the appearance of dilated capillaries and their tendency to redden. It also accelerates the skin's healing process. As it is an antioxidant, it protects the skin from free radicals, which, for example, accelerate the effect of ageing. It is also recommended for skin care after sunbathing. The numerous hydroxyl groups in the acid molecule bind significant amounts of water. In addition to its beneficial effect on the skin, the addition of lactobionic acid to a hydrogel will also increase the 'hydration' of the hydrogel.
2) D-panthenol is a provitamin B5. D-panthenol penetrates very easily into the skin, hair and nails. It penetrates the epidermis, accumulates in the dermis and changes into vitamin B5, or pantothenic acid. Applied externally, D-panthenol has a nurturing effect on the skin, accelerates cell division of skin cells, has a soothing effect on irritated and reddened skin, and is also beneficial for burned skin. A cream containing D-panthenol in its composition spreads more easily. Provitamin B5 has hygroscopic properties (can store water) and moisturises the skin. This makes the skin soft and firm. It is usually applied to the skin in concentrations of between 1% and 5%.
3) Hyaluronic acid is a chemical compound that occurs naturally in the human body, primarily in the skin, in the synovium of joints (protecting against mechanical injury and helping to distribute nutrients to cartilage), as well as in the walls of blood vessels and in the vitreous body of the eye (which is why it is sometimes added to contact lens solutions). It belongs to the group of glycosaminoglycans (GAGs) and is a component of the intercellular matrix (the space filling skin cells, which also contains the proteins of youth - collagen and elastin fibres), determining the firmness, density, smoothness and elasticity of the skin. Physiologically, hyaluronic acid is present in the skin in the form of a sodium salt (sodium hyaluronate). It has strong hygroscopic properties. In an aqueous environment, one molecule of hyaluronic acid is able to bind approximately 250 water molecules (1 g of hyaluronic acid retains approximately 6 litres of water). The addition of hyaluronic acid to the hydrogel, in addition to its beneficial effect on the skin, will also increase the 'hydration' of the hydrogel.

The result of the research is the formulation of a multicomponent hydrogel sheet, which contains active ingredients in the following concentrations:
2-3% by weight of sodium alginate,
0.1-1 by weight lactobionic acid,
0.75-5% by weight D-panthenol,
0.02-0.1% by weight of hyaluronic acid,
87.9-96.13% by weight deionised water,
1-3% by weight of calcium lactate as a crosslinking agent added in step 7 of the preparation method.

Mixtures of the composition listed above were cross-linked to form a compact hydrogel sheet using a 1-3% by weight aqueous calcium lactate solution.

Multicomponent hydrogel sheets were obtained using a method involving the following steps:
1) Weighing out the powder ingredients (sodium alginate, lactobionic acid, hyaluronic acid);
2) Mixing the ingredients in powder form for 1 minute at 100 rpm;
3) Measuring the appropriate amount of distilled water;
4) Stirring distilled water at 200 rpm and slowly adding the mixed powder ingredients (it is important that the stirrer is submerged at least halfway up the water column);
5) After adding the ingredients, the mixing speed is increased to 500 rpm with a mixing time of 20-30 minutes and after five minutes of mixing, D-panthenol is added drop by drop (at minimum concentrations of the ingredients, the total mixing time is 20 minutes, at maximum concentrations of the ingredients, the mixing time is 30 minutes);
6) An aqueous solution of calcium lactate is prepared;
7) Two sheets of blotting paper sized to match the width of the rolling rolls are soaked in the calcium lactate solution;
8) Calcium lactate-soaked blotting papers are placed between rolling rolls, a solution of the basic composition is placed between the two soaked sheets, the application of the solutions can be done with a syringe, by slow pouring from a vessel, while the spacing of the rolling rolls determines the thickness of the hydrogel sheet, the minimum value of the roll spacing is:
   for a basic mixture with a minimum composition of 1 mm;
   for a basic mixture with a maximum composition of 2 mm;
   the maximum roller pitch is:
      for a basic mixture with a minimum composition of 3 mm;
      for a basic mixture with a maximum composition of 5 mm;
9) The rolled hydrogel sheet is then immersed in a calcium lactate solution for:
   20 minutes for a base mix with a minimum composition;
   30 minutes for a base mixture of maximum composition;
10) The rolled sheet is immersed in distilled water for 5 minutes;
11) Tissue paper is removed from the sheet;
12) The edges of the sheet may be uneven, the sheet may be cut to size with scissors or another blade, or any shape may be cut from the sheet using scissors, a press or any stainless steel punch.

### Advantages of the solution:

With the method presented, hydrogel sheets with unique chemical composition (content of active ingredients), varied shape, and thickness can be obtained. The developed hydrogel formulation has not yet been presented in the state of the art, although many solutions containing sodium alginate as the basic component of the hydrogel, beads or scaffold can be found. The method for obtaining the hydrogel differs from other methods in that, due to the pre-mixing of the ingredients before solution in water, venting of the hydrogel solution, which occurs when powder ingredients are added during mixing of the hydrogel solution, is not required. In the method according to the invention, air bubbles occur in a small amount in the solution only at the first moment after the addition of the powder ingredients to the water. Furthermore, the lack of airing of the solution is also a result of immersion of the stirrer to half of the water column while maintaining the appropriate stirrer speed.

### References

1. Bouhadir K. H., Lee K. Y, Alsberg E., Damm K. L., Anderson K. W., Mooney D. J., "Degradation of Partially Oxidized Alginate and Its Potential Application for Tissue Engineering", Biotechnol. Prog. 2001, 17, 945 - 950.
2. Chen L., Shen R., Komasa S., Xue Y, Jin B., Hou Y, Okazaki J., Gao J., "Drug - Loadable Calcium Alginate Hydrogel System for Use in Oral Bone Tissue Repair", International Journal of Molecular Sciences, 2017.
3. Dubnika A., Loca D., Berzina-Cimdina L., "Functionalized hydroxyapatite scaffolds coated with sodium alginate and chitosan for controlled drug delivery", Proceedings of the Estonian Academy of Sciences, 2012, 61, 3, 193 - 199.
4. Furuya D. C., Aparecida da Costa S., Cardoso de Oliveira R., Ferraz H. G., Pessoa Junior A., Maria da Costa S., "Fibers Obtained from Alginate, Chitosan and Hybrid Used in the Development of Scaffolds", Materials Resaerch vol. 20 No. 2 (2017)
5. Garcia M. A.M., Izadifar M., Chen X., "Evaluation of PBS Treatment and PEI Coating Effects on Surface Morphology and Cellular Response of 3D - Printed Alginate Scaffolds", Journal of Functional Biomaterials 2017,8,48.
6. Li D., Li P., Zang J., Liu J., "Enhanced Hemostatic Performance of Tranexamic Acid - Loaded Chitosan/Alginate Composite Microparticles", Journal of Biomedicine and Biotechnology, Volume 2012, Article ID 981321.
7. Li Z., Ramay H. R., Hauch K. D., Xiao D., Zhang M., "Chitosan - alginate hybrid scaffolds for bone tissue engineering", Biomaterials 26 (2005) 3919 - 3928.
8. Pereira R. F., Bartolo P. J., "Degradation behaviour of biopolimer - based membranes for skin tissue regeneration", 3rd International Conference of Tissue Engineering, ICTE2013, Procedia Engineering.
9. Pereira R., Mendes A., Bartolo P., "Alginate/Aloe vera hydrogel films for biomedical applications", Procedia CIRP 5 (2013) 210-2015.
10. Qurrat-ul-Ain, Sharma S., Khuller G. K., Garg S. K., "Alginate - based oral drug delivery system for tuberculosis: pharmacokinetics and therapeutic effects", Journal of Antimicrobial Chemotherapy (2003) 51, 931 - 938.
11. Saberi A., Rafienia M., Poorazizi E., "A novel fabrication of PVA/Alginate - Bioglass electro-spun for biomedical engineering application", Nonomed.J. 2017; 4(3): 152-163.
12. Sarker B., Li W, Zheng K., Detsch R., Boccaccini A. R., "Designing Porous Bone Tissue Engineering Scafolds with Enhanced Mechanical Properties from Composite Hydrogels Composed of Modified Alginate, Gelation, and Bioactive Glass", ACS Biomater. Sci. Eng. 2016, 2, 2240 - 2254.
13. Sukhodub L. B., Yanovska G. O., Kuznetsov V. M., Martynyuk O. O., Sukhodub L. B., "Injectable Biopolymer - hydroxyapatite Hydrogels: Obtaining and their Characterization", Journal of Nano - and Electronic Physics, Vol. 8 No 1, 01032(8pp) (2016).
14. Wang Y, Wang X., Shi J., Zhu R., Zhang J., Zgang Z., Ma D., Hou Y, Lin F., Yang J., Mizuno M., "A Biomimetic Silk Fibroin/Sodium Alginate Composite Scaffold for Soft Tissue Engineering", Scientific Reports 2016.
15. Zhang X., Huang Ch., Zhao Y, Jin X., "Preparation and characterization of nanoparticle reinforced alginate fibers with high porosity for potential wound dressing application", RSC Adv., 2017, 7, 39349.

## Claims

1. A method of production of a multi-component hydrogel sheet containing:
2-3% by weight of sodium alginate,
0.1-1 by weight lactobionic acid,
0.75-5% by weight D-panthenol,
0.02-0.1% by weight of hyaluronic acid,
87.9-96.13% by weight deionised water;
1-3% by weight of calcium lactate as a crosslinking agent,
which includes the following stages:
(a) mixing sodium alginate, lactobionic acid and hyaluronic acid in powder form, preferably for 1 minute at 100 rpm;
(b) mixing with distilled water, preferably at 200 rpm, and slowly adding the mixed powder ingredients using a stirrer submerged to at least half the height of the water column;
(c) increasing the stirring speed, preferably to 500 rpm, adding D-panthenol drop by drop and stirring the whole for 15-30 minutes;
(d) soaking two sheets of blotting paper with an aqueous solution of calcium lactate and placing the soaked sheets of blotting paper between the rollers;
(e) placing the solution obtained in steps (a) to (c) between two sheets of blotting paper soaked in step (d) placed between the rollers, the rollers being spaced at between 1 and 5 mm;
(f) immersion of the tissue sheets with the solution obtained in steps (a) to (c) as a precursor to the hydrogel sheet in a calcium lactate solution for a period of 20 to 30 minutes and then in distilled water, preferably for a period of 5 minutes;
(g) removal of tissue paper sheets from the resulting hydrogel sheet.

2. A multi-component hydrogel sheet comprising:
2-3% by weight of sodium alginate,
0.1-1 by weight lactobionic acid,
0.75-5% by weight D-panthenol,
0.02-0.1% by weight of hyaluronic acid,
87.9-96.13% by weight deionised water;
1-3% by weight of calcium lactate as a crosslinking agent.

3. The multi-component hydrogel sheet according to claim 2 obtained by the method defined in claim 1.
